# EUROPÄISCHE PATENTSCHRIFT

(11) **EP 2 794 018 B1**
(45) Veröffentlichungstag und Bekanntmachung des Hinweises auf die Patenterteilung: **07.03.2018**
(21) Anmeldenummer: 12790868.9
(22) Anmeldetag: 20.11.2012
(51) Int. Cl.: A61Q 5/06, A61K 8/35, A61K 8/41, A61K 8/81, A61K 8/73

(54) **MITTEL ZUM FÄRBEN VON KERATINISCHEN FASERN ENTHALTEND KATIONISCHE ANTHRACHINONFARBSTOFFE UND ANIONISCHE POLYMERE**
SUBSTANCE FOR DYEING KERATIN FIBERS, CONTAINING CATIONIC ANTHRAQUINONE DYES AND ANIONIC POLYMERS
AGENT DE COLORATION DE FIBRES KÉRATINIQUES CONTENANT DES COLORANTS ANTHRAQUINONIQUES CATIONIQUES ET DES POLYMÈRES ANIONIQUES

(30) Priorität: 20.12.2011 DE 102011089217
(43) Veröffentlichungstag der Anmeldung: 29.10.2014
(73) Patentinhaber: Henkel AG & Co. KGaA, 40589 Düsseldorf (DE)
(72) Erfinder: WESER, Gabriele, 41472 Neuss (DE); KOLONKO, Claudia, 42857 Remscheid (DE)
(86) Internationale Anmeldenummer: PCT/EP2012/073026
(87) Internationale Veröffentlichungsnummer: WO 2013/092061

(56) Entgegenhaltungen:
- EP-A1- 1 820 826
- WO-A1-97/44004
- CA-A1- 2 613 049

## Beschreibung

Die vorliegende Erfindung betrifft Mittel zum Färben und gegebenenfalls gleichzeitigen Aufhellen von keratinischen Fasern, insbesondere menschlichen Haaren, welche kationische Anthrachinonfarbstoffe und spezielle anionische Polymere enthalten. Weiterhin betrifft die Erfindung die Verwendung dieser Mittel zur Erzeugung von Haarfärbungen mit erhöhtem Glanz, intensivem Farbergebnis, verbesserten Echtheitseigenschaften sowie verringerter Selektivität.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare Entfärbung eintritt.

Eine besondere Herausforderung für die Haarfärbung mit direktziehenden Farbstoffen stellt die gleichmäßige Färbung von häufig vorbehandeltem Haar, wie beispielsweise gebleichtem oder dauergewelltem Haar, dar, bei dem die Faser in den verschiedenen Längen oder verschieden behandelten Bereichen eine sehr unterschiedlich starke Vorschädigung besitzt. Während der Färbung selbst kann das Färbemittel auf unterschiedlich vorgeschädigtem Haar ein ungleichmäßiges Färbeverhalten zeigen, aber auch durch wiederholte Haarwäsche können die Farbstoffe in den unterschiedlichen Haarbereichen verschieden stark auswaschen werden, wodurch ein uneinheitliches und damit unerwünschtes Farbergebnis erzielt wird.

Die Herstellung von Färbeformulierungen mit verringerter Selektivität, mittels welcher auf den unterschiedlich stark geschädigten Partien der Haare ein einheitliches Farbergebnis erzielt werden kann, steht bei der Entwicklung Färbeprodukten auf Basis von Direktziehern nach wie vor besonders im Fokus. Insbesondere soll diese verringerte Selektivität nicht nur direkt nach dem Färbeprozeß, sondern auch nach wiederholten Haarwäschen noch vorhanden sein.

Die Aufgabe der vorliegenden Erfindung ist daher die Bereitstellung eines Färbemittels für keratinische Fasern, insbesondere menschliche Haare, welches neben anderen positiven Echtheitseigenschaften insbesondere eine geringe Selektivität (bzw. ein gutes Egalisiervermögen) und eine gute Waschechtheit besitzt.

Die mit den erfindungsgemäßen Mitteln erzeugten Färbungen sollen sowohl direkt nach dem Färbevorgang als auch nach wiederholten Haarwäschen ein brillantes und intensives Farbergebnis liefern. Hierbei sollen die Haare nach Anwendung des Färbemittels auch bei Vorhandensein von Haaren unterschiedlichen Schädigungsgrades gleichmäßig gefärbt sein, wobei diese Gleichmäßigkeit im Farbergebnis auch nach wiederholten Haarwäschen noch vorhanden sein soll.

Bei der vorliegenden Erfindung bestand die Zielsetzung vor allem auch darin, brillante und neutrale Blaunuancen bzw. Nuancen im Blaubereich mit den oben beschriebenen vorteilhaften Echtheitseigenschaften bereit zu stellen, welche sich hervorragend für die Mattierung eigenen. Zusätzlich sollen die Mittel eine sowohl im Hinblick auf den Anwendungsprozeß als auch auf die Färbeleistung optimierte Viskosität besitzen.

Der Einsatz von kationischen Anthrachinonfarbstoffen in Produkten zum Färben von keratinischen Fasern ist prinzipiell bereits aus dem Stand der Technik, beispielsweise aus EP 1 006 154 B1 oder aus EP 1 820 826 A1, bekannt. Ferner werden in EP 2 329 809 Kombinationen von kationischen Anthrachinonfarbstoffen mit Oxidationsfarbstoffvorprodukten vom Entwicklertyp für die oxidative Färbung von Haaren beansprucht. Aus einigen Beispielen der CA 2 613 049 A1 sind Haarfärbemittel, die bestimmte kationische Anthrachinone und anionische Polymere enthalten, bekannt. Im Zuge der zu dieser Erfindung führenden Arbeiten hat sich nun in überraschender Weise gezeigt, dass diese Kombinationen zu Färbungen führen, welche die oben beschriebene Aufgabenstellung in herausragendem Maß erfüllen.

Ein erster Gegenstand der vorliegenden Erfindung ist ein Mittel zum Färben und gegebenenfalls gleichzeitigen Aufhellen von keratinischen Fasern, insbesondere menschlichen Haaren, welches dadurch gekennzeichnet ist, dass es in einem kosmetischen Träger
(a) mindestens eine Verbindung der Formel (I) 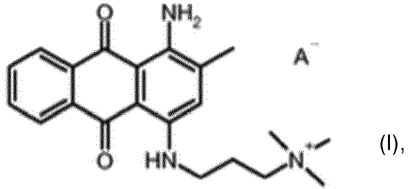 worin A für ein physiologisch verträgliches Anion, bevorzugt für Methylsulfat (H₃COSO₃⁻). steht, und
(b) mindestens ein anionisches Polymer ausgewählt aus
   (i) Polymeren der Acrylsäure und/oder der Methacrylsäure enthält.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Der erfindungsgemäß verwendete Begriff "Färben von Keratinfasern" umfasst jedwede Form der Farbveränderung der Fasern. Umfasst sind insbesondere die unter den Begriffen Tönung, Aufhellung, Blondierung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Die erfindungsgemäßen Mittel enthalten das bzw. die kationischen Anthrachinone der Formel (I) und das bzw. die anionischen Polymere in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die erfindungsgemäßen Mittel in eine pulverförmige oder auch tablettenförmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Als ersten wesentlichen Inhaltsstoff (a) enthalten die erfindungsgemäßen Mittel mindestens einen direktziehenden kationischen Anthrachinonfarbstoff der Formel (I). 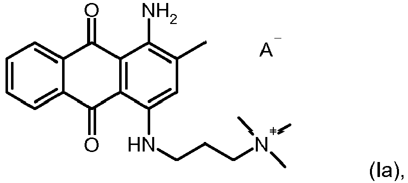 worin A⁻ für ein physiologisch verträgliches Anion, bevorzugt für Methylsulfat (H₃COSO₃⁻), steht.

Die erfindungsgemäßen Mittel zum Färben und gegebenenfalls gleichzeitigen Aufhellen von Keratinfasern enthalten die Verbindung(en) der Formel (I) vorzugsweise in Mengen oberhalb von 0,0001 Gew.-% und unterhalb von 5 Gew.-%, jeweils bezogen auf das gesamte Mittel.

Eine bevorzugte Ausführungsform ist dabei ein Mittel, welches die Verbindung(en) der Formel (I) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.

Als zweiten wesentlichen Formulierungsbestandteil (b) enthalten die erfindungsgemäßen Mittel mindestens ein anionisches Polymer ausgewählt aus (i) Polymeren der Acrylsäure und/oder der Methacrylsäure. Von der Definition Polymer mit umfasst sind sowohl die Homopolymere als auch die Copolymere der Acrylsäure und/oder Methacylsäure. Homopolmyere sind definitionsgemäß Polymere, die aus Polymeren nur einer Art entstanden sind. Im Gegensatz hierzu werden Copolymere aus mehreren verschiedenen Monomeren aufgebaut.

Die Homopolymere und Copolymere des Typs (i) zeichnen sich dadurch aus, dass sie mindestens eine Struktureinheit der Formel (P-I) enthalten, 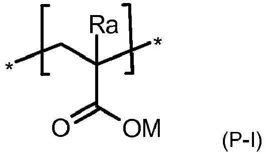 worin
- Ra: für ein Wasserstoffatom oder eine Methylgruppe steht und
- M: für ein Wasserstoffatom oder für Natrium, Kalium, ½ Magnesium oder ½ Calcium steht.

Bei einem bevorzugten Homopolymer handelt es sich um Polyacrylsäure, wie sie beispielsweise von der Firma 3V Sigma unter den Handelsnamen Synthalen K oder Synthalen M oder von der Firma Lubrizol unter den Handelsnamen Carbopol (beispielsweise Carbopol 980, 981, 954, 2984 und 5984), jeweils mit der INCI-Bezeichnung Carbomer, erhältlich ist. Auch das von der BASF vertriebene unter dem Handelsnamen Cosmedia SP (INCI Name: SODIUM POLYACRYLATE) kann in diesem Zusammenhang als bevorzugtes Acrylsäure Homopolymer genannt werden.

Auch die unter der Bezeichnung Simulgel®EG als Compound mit Isohexadecan und Polysorbat-80 vertriebenen Natriumacrylat / Natriumacryloyldimethyltaurat-Copolymere (INCI-Name: SODIUM ACRYLATE/SODIUM ACRYLOYLDIMETHYL TAURATE COPOLYMER, ISOHEXADECANE, POLYSORBATE 80) haben sich als erfindungsgemäß besonders wirksam erwiesen.

Als anionisches Polymer kann auch mindestens ein Copolymer der Acrylsäure und/oder der Methacrylsäure eingesetzt werden. Ein in diesem Zusammenhang geeignetes Polymer ist das unter der INCI Bezeichnung Acrylates / C10-30 Alkyl Acrylate Crosspolymer bekannte Polymer, das unter dem Handelsnamen Carbopol 1382 von der Firma Noveon erhältlich ist. Ein weiterhin geeignetes Polymer ist das unter der INCI-Bezeichnung Acrylates / Steareth-20 Methacrylate Crosspolymer bekannte Polymer, welches beispielsweise mit dem Handelsnamen Aculyn ® 88 von der Firma Rohm & Haas in Form einer 28 bis 30 Gew.-%-igen Dispersion in Wasser vertrieben wird. Ferner können Polymere nach der INCI-Nomenklatur als Acrylates/Palmeth-25 Acrylate Copolymer oder Acrylates / Palmeth-20 Acrylate Copolymer eingesetzt werden. Solche Polymere sind beispielsweise unter der Handelsbezeichnung Synthalen® W 2000 als 30 bis 32 Gew.-%-ige Emulsion in Wasser von der Firma 3 V Sigma erhältlich.

Innerhalb dieser Ausführungsform kann es ebenfalls bevorzugt sein, ein Copolymer aus mindestens einem anionischen Acrylsäure bzw. Methacrylsäure-Monomer und mindestens einem nichtionogenen Monomer einzusetzen. Bevorzugt nichtionogene Monomere sind in diesem Zusammenhang Acrylamid, Methacrylamid, Acrylsäureester, Methacrylsäureester, Vinylpyrrolidon, Vinylether und Vinylester.

Weitere bevorzugte anionische Copolymere sind beispielsweise Copolymere aus Acrylsäure und/oder Methacrylsäure und deren C₁-C₆-Alkylestern, wie sie unter der INCI-Deklaration Acrylates Copolymere vertrieben werden. Ein bevorzugtes Handelsprodukt ist beispielsweise Aculyn® 33 der Firma Rohm & Haas. Weiterhin bevorzugt sind aber auch Copolymere aus Acrylsäure und/oder Methacrylsäure, den C₁-C₆-Alkylestern von Acrylsäure und/oder Methacacrylsäure sowie den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn® 22 (INCI-Name: Acrylates/Steareth-20 Methacrylate Copolymer) vertrieben.

Weitere bevorzugte anionische Acrylsäure bzw. Methacrylsäure Copolymere sind Acrylsäure-Acrylamid-Copolymere.

In einer weiteren bevorzugten Ausführungsform enthält das Mittel zum Färben und gegebenenfalls gleichzeitigen Aufhellen von keratinischen Fasern als anionisches Polymer (b) ausschließlich mindestens ein Homopolymer und/oder Copolymer der Acrylsäure und/oder der Methacrylsäure. Eine weitere bevorzugte Ausführungsform ist ein Mittel zum Färben und gegebenenfalls gleichzeitigen Aufhellen von keratinischen Fasern, welches dadurch gekennzeichnet ist, dass es als anionisches Polymer (b) mindestens (i) ein Polymer der Acrylsäure und/oder der Methacrylsäure enthält, welches ausgewählt ist aus den unter den INCI-Namen bekannten Substanzen
- Carbomer (Polyacrylsäure),
- Sodium Polyacrylate,
- Sodium Acrylate/Sodium Acryloyldimethyl Taurate Copolymer,
- Acrylates / C10-30 Alkyl Acrylate Crosspolymer,
- Acrylates / Steareth-20 Methacrylate Crosspolymer,
- Acrylates/Palmeth-25 Acrylate Copolymer,
- Acrylates / Palmeth-20 Acrylate Copolymer,
- Acrylates Copolymere und/oder
- Acrylates/Steareth-20 Methacrylate Copolymer).

In einer weiteren Ausführungsform können die erfindungsgemäßen Mittel als anionisches Polymer (b) zusätzlich (ii) ein Polymer der 2-Acrylamido-2-methyl-1-propansulfonsäure enthalten. Bei den Polymeren dieser Kategorie sind ebenfalls die Homo- und Copolymere der 2-Acrylamido-2-methyl-1-propansulfonsäure von der Erfindung mit umfasst.

Polymere des Typs (ii) sind dadurch gekennzeichnet, dass sie als Strukturbestandteil mindestens eine Struktureinheit der Formel (P-II) enthalten, 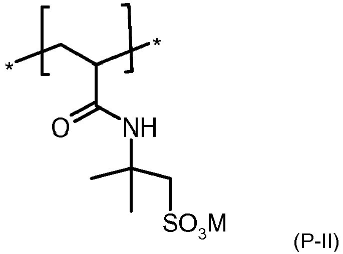 worin
- M: für ein Wasserstoffatom oder für Natrium, Kalium, ½ Magnesium oder ½ Calcium steht.

Polymere dieses Typs besitzen anionische Sulfonsäuregruppierungen, die durch Polymerisation des Monomers 2-Methyl-2-[(1-oxo-2-propenyl)amino]-1 -propansulfonsäure (AMPS) in das Polymer eingebracht werden. Als ganz besonders wirkungsvoll haben sich anionische Polymere erwiesen, die als alleiniges Monomer 2-Methyl-2[(1-oxo-2-propenyl)amino]-1 -propansulfonsäure (AMPS) enthalten, wobei die Sulfonsäuregruppe ganz oder teilweise als Natrium-, Kalium-, Ammonium-, Mono- oder Triethanolammonium-Salz vorliegen kann.

Homopolymere der 2-Methyl-2[(1-oxo-2-propenyl)amino]-1-propansulfonsäure besitzen zum Einsatz in den erfindungsgemäßen Mitteln eine gute Eignung. Als besonders geeignete Substanzen können die in der Anmeldung EP 0815828 A1 offenbarten Verbindungen, das unter dem Namen Cosmedia HSP 1160 bekannte Polymer sowie das unter der Bezeichnung Rheothik®11-80 im Handel befindliche Produkt genannt werden.

Polymere, die durch Copolymerisation des 2-Methyl-2[(1-oxo-2-propenyl)amino]-1-propansulfonsäure-Bausteins mit weiteren Monomeren erhalten werden, sind ebenfalls von der Erfindung mit umfasst und bevorzugt.

Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxythan, Allylsucrose, Allylpentaerythrit und Methylenbisacrylamid zum Einsatz kommen.

Weitere bevorzugte Sulfonsäure-Polymere dieser Art sind Copolymere aus 2-Methyl-2[(1-oxo-2-propenyl)amino]-1 -propansulfonsäure (AMPS) und Natriumacrylat, die beispielsweise als Handelsprodukt Simulgel® EG (INCI-Bezeichnung: Sodium Acrylates/Sodium Acryloyldimethyl Taurate Copolymer, Isohexadecane, Polysorbate 80) der Firma Seppic erhältlich sind.

In einer weiteren Ausführungsform können die erfindungsgemäßen Mittel als anionisches Polymer (b) zusätzlich ein (iii) anionisches Polysaccharid enthalten. In die Gruppe der (iii) anionischen Polysaccacharide fallen Xanthane, Alginate, Carboxyalkylcellulosen und Hyaluronsäuren.

Xanthan ist ein anionisches Polysaccharid, welches unter anderem aus den Strukturbestandteilen D-Glucose, D-Mannose, D-Glucuronsäure, Acetat und Pyruvat aufgebaut ist und das auch unter dem INCI Namen Xanthan Gum bekannt ist.

Als Alginate (INCI-Name Algin) werden die Salze der Alginsäure bezeichnet. Alginate sind saure, Carboxy-Gruppen enthaltende Polysaccharide, bestehend aus D-Mannuronsäure und D-Guluronsäure in unterschiedlichen Verhältnissen, welche mit 1-4-glycosidischen Bindungen verknüpft sind. Erfindungsgemäß sind sowohl die Alkali- als auch die Erdalkalisalze der Alignsäuren. Besonders vorteilhaft hat sich der Einsatz von Alginsäure, Natriumalginat, Kaliumalginat, Ammoniumalginat und/oder Calciumalginat in den erfindungsgemäßen Mitteln herausgestellt. Carboxyalkylcellulosen sind Celluloseether, bei denen die Wasserstoffatome der Hydroxygruppen der Cellulose teilweise oder vollständig durch Carboxyalkylgruppen substituiert sind. Eine bevorzugte Carboxyalkylcellulose ist die Carboxymethylcellulose, die vorzugsweise in Form ihres Natriumsalzes (Natrium-Carboxymethylcellulose) als anionisches Polymer Einsatz finden kann.

Grundbausteine der Hyaluronsäure (INCI Namen Hyaluronic acid, Sodium Hyaluronat) ist ein aus D-Glucuronsäure und N-Acetylglucsoamin in 1-3-glykosidischer Bindung aufgebautes Aminodisaccharid, das mit der nächsten Einheit β-1-4-glykosidisch verbunden ist. Natrium- und Kaliumsalze der Hyaluronsäure haben sich im Rahmen der zu dieser Erfindung führenden Arbeiten als besonders geeignet zur Erzeugung von intensiv färbenden und im Hinblick auf ihre Viskosität optimierten Färbeformulierungen erwiesen.

Eine weitere bevorzugte Ausführungsform ist ein Mittel zum Färben und gegebenenfalls gleichzeitigen Aufhellen von keratinischen Fasern, welches dadurch gekennzeichnet ist, dass es als anionisches Polymer (b) mindestens (iii) ein anionisches Polysaccharid, bevorzugt ausgewählt aus
- Xanthan (Xanthan Gum),
- Alginat (Algin),
- Carboxymethylcellulose und/oder deren physiologisch verträglichen Salzen und/oder
- Hyaluronsäure und/oder deren physiologisch verträglichen Salzen
enthält.

In einer weiteren Ausführungsform können die erfindungsgemäßen Mittel als anionisches Polymer (b) zusätzlich ein (iv) Polymer der Itaconsäure und/oder der Crotonsäure enthalten. Sowohl die Homopolymere als auch die Copolymere der Itaconsäure und/oder der Crotonsäure sind von der Erfindung mit umfasst. Polymere dieses Typs zeichnen sich dadurch aus, dass sie als Strukturbestandteile mindestens eine Einheit der Formel (P-III) und/oder der Formel (P-IV) enthalten, 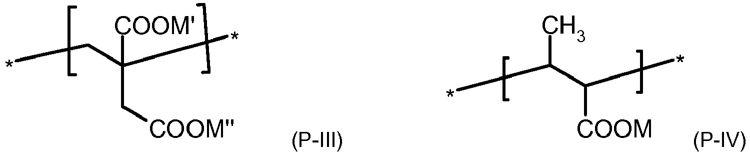 worin
- M, M', M": jeweils unabhängig voneinander für ein Wasserstoffatom oder für Natrium, Kalium, ½ Magnesium oder ½ Calcium steht.

Ein in diese Klasse gehöriges bevorzugtes Copolymer ist beispielsweise das durch die Copolymerisation von Vinylchlorid, Vinylacetat und Itaconsäure herzustellbare Terpolymer, welches auch unter dem Handelsnamen Vinnol E 15/45 M von der Firma Wacker Polymer Systems kommerziell erhältlich ist. In einer weiteren Ausführungsform können die erfindungsgemäßen Mittel als anionisches Polymer (b) zusätzlich ein (v) Polymer des Maleinsäureanhydrids enthalten. In diese Gruppe fallen Homopolymere und Copolymere enthaltend mindestens einen Strukturbestandteil der Formel (P-V), welcher durch die Polymerisation und Hydrolyse des Maleinsäureanhydrid-Monomerbausteins entsteht, 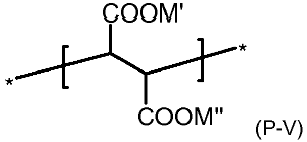 worin
- M', M": unabhängig voneinander für ein Wasserstoffatom oder für Natrium, Kalium, ½ Magnesium oder ½ Calcium stehen.

In diesem Zusammenhang bevorzugte anionische Polymere sind Copolymere aus Maleinsäureanhydrid und Methylvinylether, insbesondere solche mit Vernetzungen. Ein mit 1,9-Decadien vernetztes Maleinsäure-Methylvinylether-Copolymer ist unter der Bezeichnung Stabileze® QM im Handel erhältlich.

Die mit Einsatz dieser Polymere bzw. Polymerkombinationen herstellbaren Färbemittel zeigen im Hinblick auf die erfindungsgemäße Aufgabenstellung außerordentlich gute Eignung.

Des weiteren ist es besonders bevorzugt, wenn im erfindungsgemäßen Mittel mindestens ein anionisches Polymer (b) enthalten ist, das ausgewählt ist aus
(i) Polymeren und Copolymeren der Acrylsäure und/oder der Methacrylsäure und
(ii) Polymeren und Copolymeren der 2-Acrylamido-2-methyl-1-propansulfonsäure, da bei Zusatz der Kombinationen dieser Verbindungen besonders intensiv färbende und in ihrer Viskosität optimal eingestellte Färbeformulierungen erhalten werden können.

Ebenfalls ist es ganz besonders bevorzugt, wenn im erfindungsgemäßen Mittel mindestens ein anionisches Polymer (b) enthalten ist, das ausgewählt ist aus
(i) Polymeren und Copolymeren der Acrylsäure und/oder der Methacrylsäure und
(iii) anionischen Polysacchariden,
da bei Anwesenheit der Kombinationen dieser Verbindungen in der Färbeformulierung intensiv färbende und besonders glänzende Färbungen erhalten werden können.

Die erfindungsgemäßen Mittel zum Färben und/oder Aufhellen von keratinischen Fasern enthalten das bzw. die anionischen Polymere in einem Anteil von 0,001 bis 15 Gew.-%, bevorzugt 0,05 bis 12 Gew.-%, besonders bevorzugt 0,1 bis 10,0 Gew.-%, insbesondere 0,5 bis 5,0 Gew.-%, und insbesondere bevorzugt von 0,75 bis 3,0 Gew.-%, jeweils bezogen auf das Gesamtgewicht des Mittels.

Eine weitere bevorzugte Ausführungsform ist daher ein Mittel zum Färben und gegebenenfalls gleichzeitigen Aufhellen von keratinischen Fasern, welches dadurch gekennzeichnet ist, dass es das oder die anionischen Polymere in einem Anteil von 0,001 bis 15 Gew.-%, bevorzugt 0,05 bis 12 Gew.-%, besonders bevorzugt 0,1 bis 10,0 Gew.-%, insbesondere 0,5 bis 5,0 Gew.-%, und insbesondere bevorzugt von 0,75 bis 3,0 Gew.-%, jeweils bezogen auf das Gesamtgewicht des Mittels, enthält.

In einer weiteren bevorzugten Ausführungsform enthalten die erfindungsgemäßen Mittel neben der Verbindung der Formel (I) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17 sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)-amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Färbeergebnisse mit herausragender Farbintensität, Brillanz und guter Waschechtheit werden insbesondere dann erhalten, wenn die erfindungsgemäßen Mittel als weiteren direktziehenden Farbstoff mindestens einen Farbstoff ausgewählt aus D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxethyl)-4-methyl-2-nitroanilin (Methyl Yellow), HC Yellow No. 2, Red B 54 und 2-Amino-6-chlor-4-phenol enthalten.

Die erfindungsgemäßen Mittel können weiterhin auch als Oxidationsfärbemittel eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die direktziehenden Farbstoffe, Entwicklerkomponenten und Kupplerkomponenten werden bevorzugt jeweils in einer Menge von 0,0001 bis 5,0 Gew.-%, vorzugsweise 0,001 bis 2,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Falle von Oxidationsfärbemitteln enthalten die Mittel bevorzugt ein Oxidationsmittel, bevorzugt Wasserstoffperoxid. Die Mengen an Wasserstoffperoxid entsprechen den Mengen in den erfindungsgemäßen Aufhellmitteln.

Die Färbemittel können weiterhin als aufhellende Färbemittel eingesetzt werden. Zur Erzielung des Aufhelleffekts enthalten die Mittel dazu Wasserstoffperoxid und/oder eines seiner festen Anlagerungsprodukte an organische oder anorganische Verbindungen.

Eine weitere Ausführungsform des ersten Erfindungsgegenstands ist daher dadurch gekennzeichnet, dass das Mittel zusätzlich Wasserstoffperoxid und/oder eines seiner festen Anlagerungsprodukte an organische oder anorganische Verbindungen enthält.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Eine weitere bevorzugte Ausführungsform ist ein Mittel, zum Färben und gleichzeitigen Aufhellen keratinischer Fasern, welches zusätzlich Wasserstoffperoxid, eines seiner festen Anlagerungsprodukte an organische oder anorganische Verbindungen, Ammoniumperoxodisulfat, Kaliumperoxodisulfat und/oder Natriumperoxodisulfat jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, enthält.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten. Die anwendungsbereiten Färbemittel können weitere Hilfs- und Zusatzstoffe enthalten. So hat es sich als vorteilhaft erwiesen, wenn die Mittel mindestens ein Verdickungsmittel enthalten, welche von den Mitteln des ersten Erfindungsgegenstandes verschieden sind. Bezüglich dieser Verdickungsmittel bestehen keine prinzipiellen Einschränkungen. Es können sowohl organische als auch rein anorganische Verdickungsmittel zum Einsatz kommen.

Geeignete, von den anionischen Polymeren des ersten Erfindungsgegenstandes verschiedene Verdickungsmittel sind kationische, synthetische Polymere; natürlich vorkommende Verdickungsmittel, wie nichtionische Guargums, Skleroglucangums oder Gummi arabicum, Ghatti-Gummi, Karaya-Gummi, Tragant-Gummi, Carrageen-Gummi, Johannisbrotkernmehl, Pektine, Stärke-Fraktionen und Derivate wie Amylose, Amylopektin und Dextrine, sowie nichtionische Cellulosederivate, wie beispielsweise Methylcellulose und Hydroxyalkylcellulosen; nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Der pH-Wert der erfindungsgemäßen Mittel kann daher zwischen 3 und 11 liegen. Es ist bevorzugt, wenn der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

In einer weiteren bevorzugten Ausführungsform kann die Wirkung des erfindungsgemäßen Mittels durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten Fettsäuren mit 8 bis 22 C-Atomen,
- Sterine, wobei als Sterine eine Gruppe von Steroiden verstanden wird, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide, vor allem Glucose-Phospolipide, die z. B. als Lecithine bzw. Phosphatidylcholine aus z.B. Eidotter oder Pflanzensamen (z.B. Sojabohnen) gewonnen werden,
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls® PGPH)
- Lineare und verzweigte Fettsäuren mit 8 bis 30 C-Atomen und deren Na-, K-, Ammonium-, Ca-, Mg- und Zn-Salze.

Die erfindungsgemäßen Mittel enthalten die Emulgatoren bevorzugt in Mengen von 0,1 bis 25 Gew.-%, insbesondere 0,5 bis 15 Gew.-%, bezogen auf die Gesamtmenge des anwendungsbereiten Mittel. Nichtionogene Emulgatoren bzw. Tenside mit einem HLB-Wert von 10-15 können erfindungsgemäß besonders bevorzugt sein. Unter den genannten Emulgatoren-Typen können die Emulgatoren, welche kein Ethylenoxid und/oder Propylenoxid im Molekül enthalten ganz besonders bevorzugt sein. Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zur Anwendung der erfindungsgemäßen Mittel eignet sich insbesondere ein Verfahren zum Färben von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Die erfindungsgemäßen Mittel können als Einkomponentenmittel (Färbe- und Aufhellmittel) oder als Mehrkomponentenmittel wie Zweikomponenten Mittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Enthält das erfindungsgemäße Mittel sowohl direktziehende Farbstoffe - sowie gegebenenfalls zusätzlich Oxidationsfarbstoffvorprodukte - als auch Oxidationsmittel, so werden diese, um eine vorzeitige, unerwünschte Reaktion miteinander zu verhindern, zweckmäßigerweise getrennt voneinander konfektioniert und erst unmittelbar vor der Anwendung in Kontakt gebracht.

Ein Färbe- und Aufhellverfahren, bei dem die Färbecreme und das Oxidationsmittel zunächst getrennt vorliegen, ist daher bevorzugt. Ein weiterer Gegenstand der vorliegenden Erfindung ist daher ein Verfahren zum Färben und Aufhellen von menschlichen Haaren, bei dem eine Zusammensetzung auf wässriger Grundlage, enthaltend Wasserstoffperoxid, mit einem erfindungsgemäßen Mittel enthaltend mindestens eine Verbindung der Formel (I) zu einer homogenen Zusammensetzung vermischt, und diese auf das Haar aufgebracht wird. Das anionische Polymer (b) kann in diesem Fall sowohl zusammen mit der Wasserstoffperoxid-Lösung als auch mit der Verbindung der Formel (I) konfektioniert werden.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens einen kationischen Anthrachinonfarbstoff der Formel (I) - sowie gegebenenfalls zusätzlich Oxidationsfarbstoffvorprodukte enthält, und ein weiterer Container eine Oxidationsmittelzubereitung (B), enthaltend mindestens ein Oxidationsmittel, enthält. Das anionische Polymer (b) kann in diesem Fall sowohl zusammen mit dem kationischen Anthrachinonfarbstoff der Formel (I) in Container (A) als auch zusammen mit der Oxidationsmittelzubereitung in Container (B) konfektioniert werden.

Die Formulierung einer Kombination von (a) Verbindungen der allgemeinen Formel (I) mit (b) speziellen anionischen Polymeren eignet sich hervorragend zur Erzeugung von intensiven Färbungen mit hoher Brillanz, hohem Glanz und einer niedrigen Selektivität in Verbindung mit einer hervorragenden Waschechtheit.

Ein weiterer Gegenstand der vorliegenden Erfindung ist die Verwendung eines Mittels des ersten Erfindungsgegenstands zur Erzeugung von Haarfärbungen mit erhöhtem Glanz, intensivem Farbergebnis mit verbesserten Echtheitseigenschaften und/oder verringerter Selektivität.

Bezüglich weiterer bevorzugter Ausführungsformen der erfindungsgemäßen Verfahren und Verwendung gilt mutatis mutandis das zu den erfindungsgemäßen Mitteln Gesagte.

### Ausführunqsbeispiele

Es wurden die folgenden Rezepturen hergestellt. Die Mengenangaben verstehen sich, sofern nichts anderes vermerkt ist, jeweils in Gewichtsprozent.

### Formulierunqsbeispiel 1

| **Beschreibung** | **Gew.-%** |
|---|---|
| Coconut Alcohol | 4,00 |
| Cocamidopropyl betaine, 40% | 4,00 |
| Sodium Myreth Sulfate (2 EO), 27% | 4,00 |
| Laureth-2 | 0,80 |
| Emulgade 1000 NI | 3,00 |
| Methylparaben | 0,15 |
| Propylparaben | 0,19 |
| Polyethylene Glycol MG 400 | 3,00 |
| Acid Red 33 | 0,005 |
| N-(2-Hydroxyethyl)-4-methyl-2-nitroanilin (Methyl Yellow) | 0,04 |
| Carbomer [1000 - 7000 mPas (0,2 %)] | 0,50 |
| Monoethanolamine | 0,23 |
| 1,3-Butanediol | 1,00 |
| Water, demineralized | 3,00 |
| Polyquaternium-6 | 0,50 |
| Cationic Blue 347 | 0,20 |
| Parfum | qs |
| Wasser | Ad 100 |

### Formulierunqsbeispiel 2

| **Beschreibung** | **Gew.-%** |
|---|---|
| Methylparaben | 0,10 |
| Carbomer [20000 - 30000 mPas (0,2 %)] | 0,20 |
| KOH, 50% | 0,10 |
| Jaguar C-17 | 0,70 |
| Genamin KDMP | 3,00 |
| Cetearyl Alcohol + PEG-20 Stearate | 2,50 |
| Cetearyl Alcohol | 3,00 |
| Paraffinum Liquidum | 2,00 |
| Emulgade 1000 NI | 2,40 |
| Genamin CTAC | 3,00 |

| | |
|---|---|
| Acid violet 43 | 0,008 |
| Cationic Blue 347 | 0,20 |
| Prestige Bright Mystic Violet | 0,12 |
| Prestige Amethyst | 0,11 |
| Prestige Fire-Red | 0,012 |
| KH₂PO₄ | 0,30 |
| Benzophenone-4 | 0,10 |
| D-Panthenol, 75 % | 1,00 |
| Dimethicone / Dimethiconol | 2,00 |
| Phenoxyethanol | 0,40 |
| Crodarom Rock Crystal | 1,00 |
| Methylisothiazolone, ca. 10% in H₂O | 0,10 |
| Parfüm | qs |
| Wasser | Ad 100 |

### Rezepturbestandteile

- Cationic Blue 347: 3-[(4-Amino-9,10-dihydro-3-methyl-9,10-dioxo-1-anthracenyl)amino]-N,N,N-trimethyl-1-propanaminiummethylsulfat
- Emulgade 1000 NI: INCI-Bezeichnung: Cetearyl alcohol, Cetearath-20 (BASF)
- Jaguar C-17: INCI-Bezeichnung: Guar Hydroxypropyltrimonium Chloride (Rhodia)
- Genamin KDMP: ca. 80% Aktivgehalt; INCI-Bezeichnung: Behentrimonium Chloride, Isopropanol (Clariant)
- Genamin CTAC: ca. 29% Aktivgehalt; INCI-Bezeichnung: Aqua, Cetrimonium Chloride (Clariant)
- Emulgade 1000 NI: INCI-Bezeichnung: Cetearyl alcohol, Cetearath-20 (BASF)
- Prestige Bright Mystic Violet: INCI-Bezeichnung: Mica, CI 77491 (Iron Oxides), Tin Oxide (Eckart Cosmetic Colours)
- Prestige Amethyst: INCI-Bezeichnung: Mica, CI 77891 (Titanium Dioxide), Tin Oxide, CI 77510 (Ferric Ferrocyanide), CI 75470 (Carmine) (Eckart Cosmetic Colours)
- Prestige Fire-Red: INCI-Bezeichnung: Mica, CI 77491 (Iron Oxides) (Eckart Cosmetic Colours)
- Crodarom Rock Crystal: INCI-Bezeichnung: Aqua, Propylene Glycol, Quartz (Crodarom)

Die Färbeformulierungen wurden auf Haarsträhnen aufgetragen und dort für 30 Minuten bei Raumtemperatur belassen. Anschleißend wurden die Fasern gründlich mit Wasser ausgespült und getrocknet. Die behandelten Fasern zeichneten sich durch intensive Färbungen mit hohem Glanz und einer hervorragenden Waschechtheit aus.

## Patentansprüche

1. Mittel zum Färben und gegebenenfalls gleichzeitigen Aufhellen von keratinischen Fasern, insbesondere menschlichen Haaren, enthaltend in einem kosmetischen Träger
(a) mindestens eine Verbindung der Formel (I) 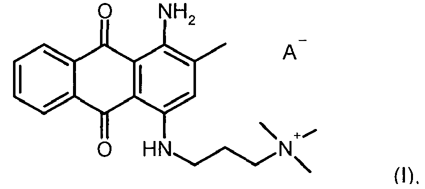 worin A⁻ für ein physiologisch verträgliches Anion, steht, und
(b) mindestens ein anionisches Polymer,
wobei das Mittel als anionisches Polymer (b) ein Polymer ausgewählt aus (i) Polymeren der Acrylsäure und/oder der Methacrylsäure enthält

2. Mittel nach Anspruch 1, **dadurch gekennzeichnet, dass** es die Verbindung(en) gemäß Formel (I) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.

3. Mittel nach einem der Ansprüche 1 bis 2, **dadurch gekennzeichnet, dass** es das oder die anionischen Polymere in einem Anteil von 0,001 bis 15 Gew.-%, bevorzugt 0,05 bis 12 Gew.-%, besonders bevorzugt 0,1 bis 10,0 Gew.-%, insbesondere 0,5 bis 5,0 Gew.-%, und insbesondere bevorzugt von 0,75 bis 3,0 Gew.-%, jeweils bezogen auf das Gesamtgewicht des Mittels, enthält.

4. Mittel nach einem der Ansprüche 1 bis 3, **dadurch gekennzeichnet, dass** es als anionisches Polymer (b) mindestens (i) ein Polymer der Acrylsäure und/oder der Methacrylsäure enthält, welches ausgewählt ist aus den unter den INCI-Namen bekannten Substanzen
- Carbomer (Polyacrylsäure),
- Sodium Polyacrylate,
- Sodium Acrylate/Sodium Acryloyldimethyl Taurate Copolymer,
- Acrylates / C10-30 Alkyl Acrylate Crosspolymer,
- Acrylates / Steareth-20 Methacrylate Crosspolymer,
- Acrylates/Palmeth-25 Acrylate Copolymer,
- Acrylates / Palmeth-20 Acrylate Copolymer,
- Acrylates Copolymere und/oder
- Acrylates/Steareth-20 Methacrylate Copolymer).

5. Mittel nach einem der Ansprüche 1 bis 4, **dadurch gekennzeichnet, dass** es als anionisches Polymer (b) zusätzlich mindestens (iii) ein anionisches Polysaccharid, bevorzugt ausgewählt aus
- Xanthan (Xanthan Gum),
- Alginat (Algin),
- Carboxymethylcellulose und/oder deren physiologisch verträglichen Salzen und/oder
- Hyaluronsäure und/oder deren physiologisch verträglichen Salzen enthält.

6. Mittel nach einem der Ansprüche 1 bis 5, **dadurch gekennzeichnet, dass** es neben der Verbindung der Formel (I) zusätzlich mindestens einen weiteren direktziehenden Farbstoff enthält.

7. Mittel nach Anspruch 6, **dadurch gekennzeichnet, dass** es als zusätzlichen direktziehenden Farbstoff mindestens einen Farbstoff aus der Gruppe D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxethyl)-4-methyl-2-nitroanilin (Methyl Yellow), HC Yellow No. 2, Red B 54 und 2-Amino-6-chlor-4-phenol enthält.

8. Mittel nach einem der Ansprüche 1 bis 44 7, **dadurch gekennzeichnet, dass** es zusätzlich Wasserstoffperoxid und/oder eines seiner festen Anlagerungsprodukte an organische oder anorganische Verbindungen enthält.

9. Mittel nach einem der Ansprüche 1 bis 42 8, **dadurch gekennzeichnet, dass** es zusätzlich mindestens ein zwitterionisches Tensid ausgewählt aus den N-Alkyl-N,N-dimethylammoniumglycinaten, den N-Acyl-aminopropyl-N,N-dimethylammoniumglycinaten und den 2-Alkyl-3-carboxymethyl-3-hydroxyethyl-imidazolinen enthält.

10. Mittel nach Anspruch 9, **dadurch gekennzeichnet, dass** es zusätzlich als zwitterionisches Tensid Cocamidopropyl Betaine enthält.

11. Verwendung eines Mittels nach einem der Ansprüche 1 bis 10 zur Erzeugung von Haarfärbungen mit verringerter Selektivität.

## Claims

1. An agent for dyeing and optionally simultaneously lightening keratin fibers, in particular human hair, containing, in a cosmetic carrier,
(a) at least one compound of formula (I), 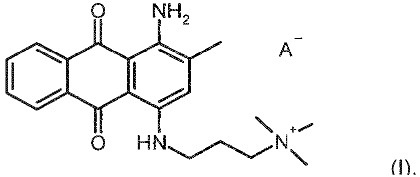 wherein A⁻ represents a physiologically acceptable anion,
and
(b) at least one anionic polymer,
wherein the agent contains, as the anionic polymer (b), a polymer selected from (i) polymers of acrylic acid and/or methacrylic acid.

2. The agent according to claim 1, **characterized in that** it contains the compound(s) according to formula (I) in a proportion of from 0.0001 to 5 wt.%, preferably from 0.005 to 3.5 wt.%, particularly preferably from 0.01 to 2.5 wt.%, in particular from 0.05 to 1.5 wt.%, and in particular preferably from 0.01 to 1.0 wt.%, in each case based on the total weight of the agent.

3. The agent according to one of claims 1 to 2, **characterized in that** it contains the anionic polymer(s) in a proportion of from 0.001 to 15 wt.%, preferably from 0.05 to 12 wt.%, particularly preferably from 0.1 to 10.0 wt.%, in particular from 0.5 to 5.0 wt.%, and in particular preferably from 0.75 to 3.0 wt.%, in each case based on the total weight of the agent.

4. The agent according to one of claims 1 to 3, **characterized in that** it contains, as the anionic polymer (b), at least (i) one polymer of acrylic acid and/or methacrylic acid, which polymer is selected from the substances known under the INCI names:
- Carbomer (polyacrylic acid),
- Sodium Polyacrylate,
- Sodium Acrylate/Sodium Acryloyldimethyl Taurate Copolymer,
- Acrylates / C10-30 Alkyl Acrylate Crosspolymer,
- Acrylates / Steareth-20 Methacrylate Crosspolymer,
- Acrylates/Palmeth-25 Acrylate Copolymer,
- Acrylates / Palmeth-20 Acrylate Copolymer,
- Acrylates Copolymer and/or
- Acrylates/Steareth-20 Methacrylate Copolymer.

5. The agent according to one of claims 1 to 4, **characterized in that** it additionally contains, as the anionic polymer (b), at least (iii) one anionic polysaccharide, preferably selected from
- xanthan gum,
- algin,
- carboxymethylcellulose and/or physiologically acceptable salts thereof and/or
- hyaluronic acid and/or physiologically acceptable salts thereof.

6. The agent according to one of claims 1 to 5, **characterized in that** it additionally contains, in addition to the compound of formula (I), at least one further substantive dye.

7. The agent according to claim 6, **characterized in that** it contains, as the additional substantive dye, at least one dye from the group of: D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-hydroxyethyl)-4-methyl-2-nitroaniline (Methyl Yellow), HC Yellow No. 2, Red B 54 and 2-amino-6-chloro-4-phenol.

8. The agent according to one of claims 1 to 7, **characterized in that** it additionally contains hydrogen peroxide and/or one of the solid addition products of addition thereof to organic or inorganic compounds.

9. The agent according to one of claims 1 to 8, **characterized in that** it additionally contains at least one zwitterionic surfactant selected from the N-alkyl-N,N-dimethylammonium glycinates, the N-acyl-aminopropyl-N,N-dimethylammonium glycinates and the 2-alkyl-3-carboxymethyl-3-hydroxyethyl-imidazolines.

10. The agent according to claim 9, **characterized in that** it additionally contains cocamidopropyl betaines as the zwitterionic surfactant.

11. The use of an agent according to one of claims 1 to 10 for producing hair dyes having reduced selectivity.

## Revendications

1. Agent de coloration et le cas échéant, simultanément, d'éclaircissement de fibres kératiniques, notamment de cheveux humains, contenant dans un support cosmétique
(a) au moins un composé de la formule (I) 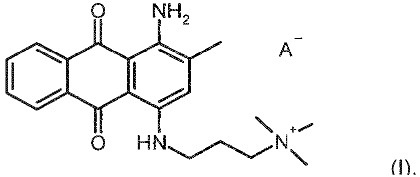 dans laquelle A représente un anion physiologiquement compatible
et
(b) au moins un polymère anionique,
dans lequel l'agent contient en tant que polymère (b) un polymère sélectionné parmi (i) les polymères de l'acide acrylique et/ou de l'acide méthacrylique.

2. Agent selon la revendication 1, **caractérisé en ce qu'**il contient le(s) composé(s) selon la formule (I) dans une proportion de 0,0001 à 5 % en poids, de manière préférée 0,005 à 3,5 %, de manière particulièrement préférée 0,01 à 2,5 % en poids, notamment 0,05 à 1,5 % en poids et de manière préférée entre toutes de 0,01 à 1,0 % en poids, respectivement rapporté au poids total de l'agent.

3. Agent selon l'une quelconque des revendications 1 à 2, **caractérisé en ce qu'**il contient le ou les polymères anioniques dans une proportion de 0,001 à 15 % en poids, de manière préférée 0,05 à 12 % en poids, de manière particulièrement préférée 0,1 à 10,0 % en poids, notamment 0,5 à 5,0 % en poids et de manière préférée entre toutes de 0,75 à 3,0 % en poids, respectivement rapporté au poids total de l'agent.

4. Agent selon l'une quelconque des revendications 1 à 3, **caractérisé en ce qu'**il contient, en tant que polymère anionique (b), au moins (i) un polymère de l'acide acrylique et/ou de l'acide méthacrylique, lequel est sélectionné parmi les substances connues sous les dénominations INCI
- Carbomer (acide polyacrylique),
- Sodium Polyacrylate,
- Sodium Acrylate/Sodium Acryloyldimethyl Taurate Copolymer,
- Acrylates / C10-30 Alkyl Acrylate Crosspolymer,
- Acrylates / Steareth-20 Methacrylate Crosspolymer,
- Acrylates/Palmeth-25 Acrylate Copolymer,
- Acrylates / Palmeth-20 Acrylate Copolymer,
- Acrylates Copolymere et/ou
- Acrylates/Steareth-20 Methacrylate Copolymer).

5. Agent selon l'une quelconque des revendications 1 à 4, **caractérisé en ce qu'**il contient, en tant que polymère anionique (b), de plus au moins (iii) un polysaccharide anionique, préférablement sélectionné parmi
- la xanthane (Xanthan Gum),
- l'alginate (Algin),
- la carboxyméthylcellulose et/ou ses sels physiologiquement compatibles et/ou
- l'acide hyaluronique et/ou ses sels physiologiquement compatibles.

6. Agent selon l'une quelconque des revendications 1 à 5, **caractérisé en ce qu'**il contient, outre le composé de la formule (I), de manière supplémentaire au moins un autre colorant à action directe.

7. Agent selon la revendication 6, **caractérisé en ce qu'**il contient, en tant que colorant à action directe supplémentaire, au moins un colorant issu du groupe constitué du D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxyethyl)-4-methyl-2-nitroanilin (Methyl Yellow), HC Yellow No. 2, Red B 54 et 2-amino-6-chlor-4-phenol.

8. Agent selon l'une quelconque des revendications 1 à 7, **caractérisé en ce qu'**il contient de plus du peroxyde d'hydrogène et/ou un de ses produits d'addition solides sur des composés organiques ou inorganiques.

9. Agent selon l'une quelconque des revendications 1 à 8, **caractérisé en ce qu'**il contient de plus au moins un tensioactif zwitterionique sélectionné parmi les glycinates de n-alkyl-n,n-diméthylammonium, les glycinates de n-acyl-aminopropyl-n,n-diméthylammonium et les 2-alkyl-3-carboxyméthyl-3-hydroxyéthyl-imidazolines.

10. Agent selon la revendication 9, **caractérisé en ce qu'**il contient de plus de la Cocamidopropyl Betaine en tant que tensioactif zwitterionique.

11. Utilisation d'un agent selon l'une quelconque des revendications 1 à 10 pour la création de colorations capillaires avec une sélectivité réduite.
